# EUROPEAN PATENT APPLICATION

(11) **EP 4 131 281 A1**
(43) Date of publication of application: **08.02.2023**
(21) Application number: 21189986.9
(22) Date of filing: 05.08.2021
(51) Int. Cl.: G16H 30/20, G16H 30/40, G16H 50/70

(54) **DETERMINING A BODY REGION REPRESENTED BY MEDICAL IMAGING DATA**

(71) Applicant: Siemens Healthcare GmbH, 91052 Erlangen (DE)
(72) Inventor: SHINAGAWA, Yoshihisa, Downingtown, PA 19335 (US); YEREBAKAN, Halid, Malvern, PA 19355 (US); HERMOSILLO VALADEZ, Gerardo, West Chester, PA 19382 (US); ALLEN-RAFFL, Simon, West Chester, PA 19382 (US); RANGANATH, Mahesh, Malvern, PA 19355 (US)
(74) Representative: EIP

(57) **Abstract**

A computer implemented method and apparatus determines a body region represented by medical imaging data stored in a first image file. The first image file further stores one or more attributes each having an attribute value comprising a text string indicating content of the medical imaging data. One or more of the text strings of the first image file are obtained and input into a trained machine learning model, the machine learning model having been trained to output a body region based on an input of one or more such text strings. The output from the trained machine learning model is obtained thereby to determine the body region represented by the medical imaging data. Also disclosed are methods of selecting one or more sets of second medical imaging data as relevant to first medical imaging data.

## Description

### Technical Field

The present invention relates to a method and apparatus for determining a body region represented by medical imaging data stored in an image file.

### Background

Medical imaging, such as Magnetic Resonance Imaging (MRI), Computed Tomography (CT) and the like, is an invaluable tool for medical diagnosis. In clinical decision making, the progression of a patient's disease over time can be as, if not more, important to diagnosis than the current status of that disease. In order to help assess the progression of a patient's disease, medical professionals often wish to compare a current medical image of the patient with an appropriate previous medical image or series of medical images of the patient.

However, there are often large numbers of the previous medical images for a patient. For example, for any given patient, there may exist multiple previous studies taken at multiple previous times. Moreover, within each study, there may be multiple series of medical images each having different characteristics. Manually assessing the appropriateness of the previous medical images for comparison with, or otherwise their relevance to, the current medical image can be time consuming and burdensome for the medical professional. Further, the medical images are often stored in a storage remote from the medical professional's terminal and retrieving all of the prior medical images for a patient, which are often large in size, for the medical professional to asses is network resource intensive.

It would be useful to automatically select medical images (e.g. previous medical images) that are appropriate for comparison with or otherwise relevant to a given medical image (e.g. a current medical image).

Alternatively or additionally (e.g. in order to facilitate selection of relevant medical images or for other reasons) it would be useful to automatically determine the body region represented by a medical image. However, analyzing medical imaging data representing the image to determine the body region represented thereby would involve extraction and processing of the medical imaging data, which is typically large and would be resource intensive.

### Summary

According to a first aspect of the present invention, there is provided a computer implemented method of determining a body region represented by medical imaging data stored in a first image file, the first image file further storing one or more attributes each having an attribute value comprising a text string indicating content of the medical imaging data, the method comprising: (a)obtaining one or more of the text strings of the first image file; and (b)inputting the obtained one or more text strings into a trained machine learning model, the machine learning model having been trained to output a body region based on an input of one or more such text strings, and obtaining the output from the trained machine learning model thereby to determine the body region represented by the medical imaging data.

Optionally, the method comprises: comparing a first body region represented by first medical imaging data stored in the first image file with each of a plurality of second body regions represented by a respective plurality of sets of second medical imaging data stored in a respective plurality of second image files, each second image file further storing one or more attributes each having an attribute value comprising a text string indicating content of the second medical imaging data stored in the second image file; and selecting one or more of the sets of second medical imaging data as relevant to the first medical imaging data based on the comparison of the body regions; wherein the first body region is determined by applying steps (a) and (b) to the one or more text strings of the first image file; and/or wherein at least one of the second body regions is determined by applying steps (a) and (b) to the one or more text strings of a respective at least one of the second image files.

Optionally, the plurality of sets of second medical imaging data are stored in a remote storage device, and the method comprises: retrieving the selected one or more sets of second medical imaging data, or one or more sets of the second medical imaging data of a study including the one or more selected sets of second medical imaging data, from the remote storage without retrieving other ones of the plurality of sets of second medical imaging data.

Optionally, the method comprises: generating display data to cause a display device to display a rendering of the first medical imaging data and a rendering of one or more of the selected or retrieved sets of second medical imaging data.

Optionally, the method comprises: determining, for each of the plurality of sets of second medical imaging data, an imaging modality relevance score between a first imaging modality of the first medical imaging data and a second imaging modality of the second medical imaging data; and selecting the one or more sets of second medical imaging data as relevant to the first medical imaging data is further based on the determined imaging modality relevance score.

Optionally, the imaging modality relevance score is determined using an imaging modality transition matrix, wherein each element of the imaging modality transition matrix represents a respective probability, determined based on statistical analysis of logged user interaction with medical imaging data, that given first medical imaging data associated with a particular first imaging modality a user will select for comparison with the first medical imaging data second medical imaging data having a particular second imaging modality.

Optionally, each of the first and second image files stores one or more attributes each having an attribute value indicative of an imaging parameter used to capture the medical imaging data of the image file, and the method comprises: for each of a plurality of sets of the second medical imaging data, determining a similarity metric indicative of the similarity between a first vector, generated based on one or more of the attribute values indicative of an imaging parameter used to capture the first medical imaging data, and a second vector generated based on one or more of the attribute values indicative of an imaging parameter used to capture the second medical imaging data; and selecting the one or more sets of second medical imaging data as relevant to the first medical imaging data is further based on the determined similarity metric.

Optionally, the trained machine learning model is a trained neural network.

Optionally, the trained neural network comprises a trained character-based neural network configured to take as input individual characters of the obtained one or more text strings, whereby inputting the obtained one or more text strings into the trained neural network comprises inputting individual characters of the obtained one or more text strings into the trained character-based neural network.

Optionally, the trained neural network is configured to output the body region in the form of one or more numerical values, representing a region of a human body, that a regressor portion of the trained neural network has calculated for the input one or more text strings.

Optionally, the neural network has been trained using a training data set comprising a plurality of training text strings, each training text string being from an attribute value of an attribute of an image file and indicating content of medical imaging data further stored in the image file, each of the plurality of training text strings being labelled with a body region to which the training text string corresponds, the label being used a supervisory signal in the training of the neural network.

Optionally, the training data set has been generated using a Graphical User Interface, GUI, configured to: present one or more of the training text strings and a representation of a human body divided into selectable body regions to a user; and for each of the one or more presented training text strings, receive a user input selecting a body region from the representation, and label the training text string with a label indicating the selected body region.

Optionally, the trained neural network is further trained to output a laterality of the body region based on the input of the one or more text strings, and step (b) further comprises determining the laterality of the body region represented by the medical imaging data based on the obtained output of the trained neural network.

Optionally, the or each of the image files is a DICOM file, and the one or more attributes having attribute values comprising text strings indicating content of the medical imaging data thereof comprise one or more of the DICOM attributes 'Study Description', 'Series Description' and 'Body Part Examined'.

According to a second aspect of the present invention there is provided apparatus configured to perform the method according to the first aspect.

According to a third aspect of the present invention there is provided a computer program which, when executed by a computer, causes the computer to perform the method according to the first aspect.

Further features and advantages of the invention will become apparent from the following description of examples of the invention, given by way of example only, which is made with reference to the accompanying drawings.

### Brief Description of the Drawings

Figure 1 is a flow diagram illustrating a method according to an example;
Figure 2 is a schematic diagram illustrating an image file comprising medical imaging data according to an example;
Figure 3 is a schematic diagram illustrating a Digital Imaging and Communications in Medicine (DICOM) file comprising medical imaging data according to an example;
Figure 4 is a schematic diagram illustrating a flow between components according to an example;
Figure 5 is a schematic diagram illustrating a Graphical User Interface (GUI) according to an example;
Figure 6 is a flow diagram illustrating a method according to an example;
Figure 7 is a schematic diagram illustrating a flow between components according to an example;
Figure 8 is a flow diagram illustrating a method according to an example;
Figure 9 is a schematic diagram illustrating a flow between components, according to an example;
Figure 10 is a schematic diagram illustrating a system according to an example; and
Figure 11 is a schematic diagram illustrating a computer according to an example.

### Detailed Description

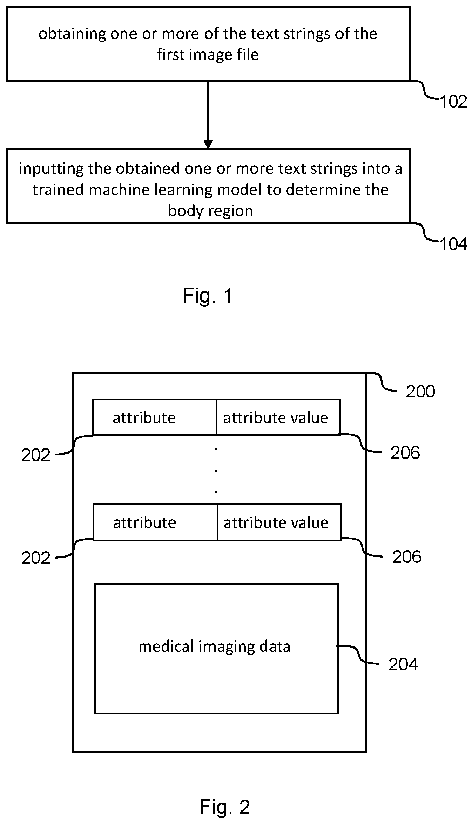

Referring to Figure 1, there is illustrated a computer implemented method of determining a body region represented by medical imaging data stored in a first image file.

An example first image file is illustrated in Figure 2. The first image file 200 stores the medical imaging data 204, and further stores one or more attributes 202 each having an attribute value 206 comprising a text string indicating content of the medical imaging data 204.

The medical imaging data 204 is data that represents a medical image (or in certain examples more than one medical image). For example, the medical imaging data 204 may comprise an array or list of pixel or voxel values. For example, when processed by suitable image viewing software, the medical imaging data results in a rendering of the medical image (or medical images) that it represents. The one or more attribute values are separate to and distinct from the medical imaging data, and instead comprise a text string indicating content of the medical imaging data 204. Such attribute values may, in some examples, be referred to as metadata of the image file 200. In some examples, the part of the image file 200 that stores the attributes 202 and attribute values 206 may be referred to as a header of the image file 200, and the attributes 202 and attribute values 206 may be referred to as header data of the image file 200.

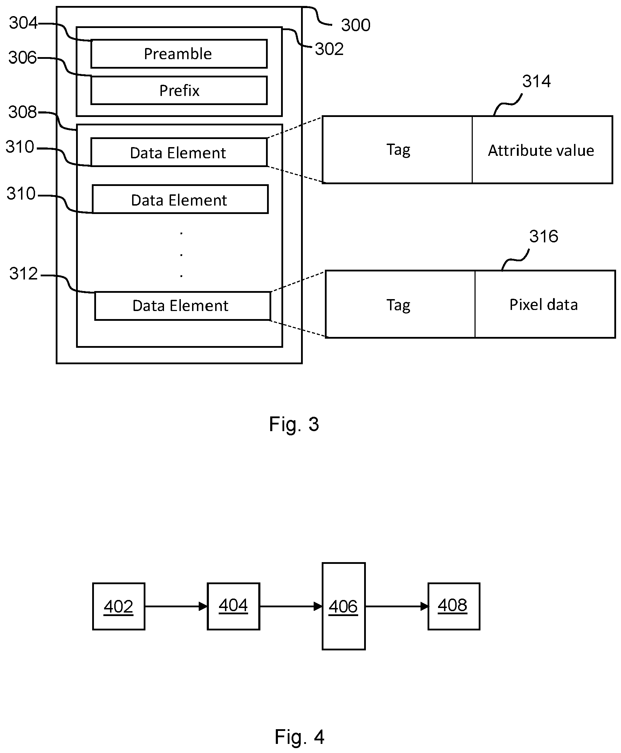

A specific example of an image file 200 is a Digital Imaging and Communications in Medicine (DICOM) file 300. An example DICOM file is illustrated in Figure 3, as is described in more detail below. In overview, the DICOM file 300 stores medical imaging data 316 as pixel data in a designated data element 312, and further stores, as one or more other data elements 310, one or more attributes 310 each having an attribute value 314 comprising a text string indicating content of the medical imaging data 316. An example such DICOM attribute 310 is 'Study Description' whose attribute value 314 is a text string that describes the study of which the medical imaging data is part (e.g. 'NERUO^HEAD' where the medical imaging data is of the head region of the patient) and thereby indicates the content of the medical imaging data 316. There are other example such DICOM attributes, such as 'Series description', 'Body Part Examined' as well as others.

Returning to Figure 1, in broad overview, the method comprises:
(a) in step 102, obtaining one or more of the text strings 206, 314 of the first image file 200, 300; and
(b) in step 104, inputting the obtained one or more text strings 206, 314 into a trained machine learning model (see e.g. the trained neural network 406 of Figure 4), the machine learning model 406 having been trained to output a body region based on an input of one or more such text strings, and obtaining the output from the trained machine learning model 406 thereby to determine the body region 408 represented by the medical imaging data 204, 316.

Determining the body region 408 represented by the medical imaging data 204, 316 by inputting the one or more text strings 206, 314 of the first image file 200, 300 into a machine learning model 406 (e.g. a neural network) trained to determine a body region based on input such text strings, may provide for efficient and/or flexible determination of the body region 408 represented by the medical imaging data.

For example, determining the body region based on the text strings of the file (which are relatively small in terms of bits) may be less resource intensive and hence more efficient than, for example, determining the body region by extracting and analyzing the medical imaging data itself (which is relatively large in terms of bits). In cases where the image file is stored remotely from a processing device over a network, determining the body region based on the (relatively small) text strings allows that the (relatively large) medical imaging data need not be transmitted over the network in order to determine the body region represented thereby, hence making efficient use of network resources.

As another example, determining the body region by inputting the text strings into a trained machine learning model (e.g. trained neural network) may provide for efficient, flexible and/or robust determination of the body region, for example as compared to determining the body region by applying hard coded rules to the text strings. For example, hard coded rules require an exact match of the text string to a rule in order to provide a body region (and hence are inflexible with respect to text strings for which a match can be determined and/or are inefficient in the exhaustive nature of the set of rules needed to be coded for all possible text strings that could be used). On the other hand, a trained machine learning model (e.g. a trained neural network) generalizes from a training data set on which it is trained, and hence is both relatively efficient to obtain and is able to determine an appropriate body region even for text strings different from those in the training data set, and hence is relatively flexible/robust.

Accordingly, efficient and/or flexible automated determination of the body region represented by medical imaging data may be provided for.

In some examples, at a given time and for a given patient, a radiologist may perform a medical imaging study on the patient. Different studies may be performed at different times. A particular study may be for a particular body part of the patient and using a particular imaging modality (e.g. MR). In some cases, different studies may be of different body parts of the patient and/or have different imaging modalities (i.e. captured by different equipment). A given study may comprise one or more series of medical images. For example, within a given series within a study, the medical images may have been captured using the same imaging parameters (e.g. patient orientation, MR specific imaging parameters such as Echo Time and the like). Each series within a study may have different imaging parameters. In some examples, an image file 200 stores medical imaging data 204 representing an image within a particular series and within a particular study. Image files 200 that store medical imaging data 204 representing medical images that are part of the same study may, for example, have the same unique study ID, and image files 200 that store medical imaging data 204 representing medical images that are part of the same series may for example, have the same unique series ID. In any case, a given study may have at least one series of at least one such image files 200.

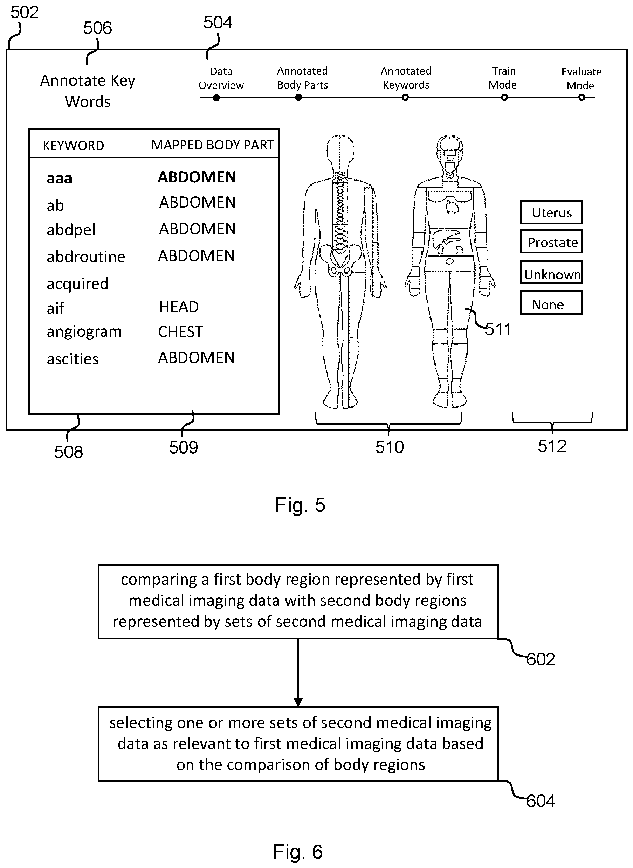

In some examples, the determination of the body region represented by first medical imaging data of the first image file 200 may in turn facilitate the automated selection of second medical imaging data of second such files as relevant (e.g. appropriate for comparison with) to the first medical imaging data. For example, determining the body region represented by the medical imaging data of a file of a current study may be used to select medical imaging data of a previous study of the same body region of the patient. This is described in more detail below with reference to Figures 4 and 5.

As mentioned above, in some examples the first image file 200, 300 may be a DICOM file 300 (i.e. an image file in a DICOM file format, e.g. according to the DICOM standard "NEMA PS3 / ISO 12052, Digital Imaging and Communications in Medicine (DICOM) Standard, National Electrical Manufacturers Association, Rosslyn, VA, USA"). Referring again to Figure 3, in more detail, the DICOM file 300 comprises a header 302 and a dataset 308. The header 302 comprises a 128 byte preamble 304 (which if not being used has all bytes set to zero) and a 4 byte prefix 306 containing the character string "DICM". The dataset 308 contains data elements 310, 312. Each data element 310, 312 comprises, and is identified by, a Tag. Each Tag is in the format (XXXX,XXXX) where each 'X' is a Hexadecimal number. The DICOM file 300 may store the unique identifier of the study of which the file is part in the 'Study ID' data element (not shown) (i.e. identified by the DICOM Tag (0020, 0010)), and may store the unique identifier of the series of which the file is part in the 'Series ID' data element (not shown) (i.e. identified by the DICOM Tag (0020,000E).

The DICOM file 300 stores medical imaging data 316 (in this case pixel data 316) in the 'pixel data' data element 312 (i.e. identified by the DICOM Tag (7FE0, 0010)) of the DICOM file 300. The DICOM file 300 further stores one or more attributes 310 (provided by one or more other data elements 310) each having an attribute value 314 comprising a text string indicating content of the medical imaging data 316. For example, one such attribute may be 'Study Description' (i.e. identified by DICOM Tag (0008, 1030)) whose attribute value 314 is a text string that describes the study of which the medical imaging data is part (e.g. 'NERUO^HEAD' where the medical imaging data is of the head region of the patient or 'PELVIS^PROSTATE' where the medical imaging data is of the pelvis region of the patient) and thereby indicates the content of the medical imaging data 316. In some examples, although the attributes 310 and attribute values 314 may not be included in the header 302 as such but rather in the dataset 308, the attributes 310 and attribute values 314 (not including the data element 312 storing the medical imaging data 316 itself) may sometimes be referred to as header data of the DICOM file 300, as these are data relating to the medical imaging data 316 rather than being the medical imaging data 316 itself.

Other example DICOM attributes which have attribute values comprising text strings indicating content of the medical imaging data may be used. For example, another such example DICOM attribute is 'Series description' (i.e. identified by DICOM Tag (0008, 103E)) which describes the series of which the medical imaging data is part (e.g. 'ax t1 whole pelvis' indicating that the medical imaging data is of the whole pelvis, was captured with an axial orientation and using T1 type MRI). For example, another such example DICOM attribute is 'Body Part Examined' (i.e. identified DICOM Tag (0018,0015)), which indicates the body part examined (e.g. 'PELVIS' indicating that the medical imaging data is of the pelvis). It will be appreciated that other such suitable DICOM attributes exist and may be used. In some examples, the DICOM attribute 'Reason for the Requested Procedure' (i.e. identified by DICOM Tag (0040, 1002)) may also be used.

Further, it will be appreciated that in some examples such image files other than DICOM files may be used. For example, in some example the image file may be a Portable Network Graphics (PNG) file storing in one of its chunks medical imaging data and in another of its chunks metadata comprising one or more attributes each having an attribute value comprising a text string indicating content of the medical imaging data.

In some examples, one such text string may be input into the trained machine learning model (e.g. trained neural network). However, in other examples, multiple such text strings from multiple attribute values of multiple attributes of the first image file may be input together into the trained machine learning model (e.g. trained neural network) in order to determine the body part represented by the medical imaging data of the first file. For example, this may provide for more accurate and/or robust determination of the body region. For example, the accuracy with which the trained machine learning model (e.g. trained neural network) determines the body region may be improved by increased input data (number of such text strings from the first file) on which the determination is based. As another example, if one of the text strings happens to be unreliable and/or missing (e.g. as it has been found can be the case for e.g. 'Body Part Examined' because it is not routinely or reliably filled out by radiologists), then the input of multiple text strings may mitigate this and still allow for a reliable determination of the body region to be determined.

Figure 4 illustrates a flow between components of the method described above with reference to Figures 1 to 3, according to an example. Referring to Figure 4, as mentioned, one or more of the text strings 404 of the first image file 402 are input into a trained machine learning 406, and the trained machine learning model 406 outputs a body region 408. The text strings 404, image file 402, trained machine learning model 406 and/or the body region 408 may be the same or similar to, and/or may be used as, those described above with reference to Figures 1 to 3.

In some examples, as illustrated, the one or more text strings 404 may be extracted from the first image file 402. Specifically, the one or more text strings 404 may be extracted from the first image file 402 without extracting the medical imaging data thereof. For example, taking the DICOM file 300 as an example, the image file may be parsed to locate one or more predefined Tags identifying the attributes having attribute values to be extracted (e.g. DICOM Tag (0008, 1030) for the 'Study Description' attribute and DICOM Tag (0008, 103E)for the 'Series Description'). Once the Tags are located, the attribute values (i.e. the text strings 404) of the one or more attributes identified by the one or more Tags may be extracted from the image file 402. The extracted text strings 404 may be stored, for example in association with an identifier for the image file 402 or for the medical imaging data thereof. In some examples, the text strings 404 of the image file 402 may have been extracted from the image file 402 or otherwise obtained in advance of the method being performed, and e.g. stored in association with an identifier for the image file 402 or for the medical imaging data thereof. In either case, the one or more text strings 404 are obtained and input into the trained machine learning model 406.

In some examples, only one text string may be used. However, in some examples, a plurality of the text strings 404 of an image file 402 may be obtained and input into the trained machine learning model 406. For example, the text strings of the attribute values of all of the attributes 'Study Description', 'Series Description', and 'Body Part Examined' of a DICOM file 402 may be obtained and input into the trained machine learning model 406 so that the trained machine learning model 406 outputs the body region represented by the medical imaging data of that file based on all of these input text strings. For example, the text strings may be concatenated and input into the trained machine learning model 406 together. In any case, the output 408 from the trained machine learning model 406 is obtained, thereby to determine the body region 408 represented by the medical imaging data.

As mentioned, the machine learning model 406 has been trained to output a body region based on an input of one or more such text strings. That is, the machine learning model is trained to output a body region represented by medical imaging data of an image file based on an input of one or more text strings, indicating content of the medical imaging data, of one or more attribute values of one or more attributes of the image file.

In some examples, the trained machine learning model 406 may be a trained neural network 406. Indeed, in the examples described hereinafter, a trained neural network 406 will be referred to. However, it will be appreciated that in other examples, other types of machine learning models trained to output a body region based on an input of one or more text strings may be used. For example, in other examples the trained machine learning model may take the form of a trained random forest algorithm or the like. For example, the random forest algorithm may comprise an ensemble of decision trees trained on training data including training text strings labelled with their associated body region (e.g. as determined by an expert), i.e. to configure the ensemble of decision trees so as to minimize the error in the prediction of the body region as compared to the labelled body region for the training text strings. However, as mentioned, hereinafter the example of a trained neural network 406 is referred to. The use of a trained neural network 406 may have certain advantages, as discussed in more detail below.

In some examples, the neural network 406 may be a deep neural network (i.e. with one or more hidden layers). In some examples, the neural network may be trained using supervised learning. For example, the neural network 406 may be trained using a training data set comprising a plurality of training text strings (in practice there may be 100s or 1000s of training text strings), each training text string being from an attribute value of an attribute of an image file and indicating content of medical imaging data further stored in the image file. For example, the training text strings may be text strings extracted from attribute values of appropriate attributes of a DICOM image file (e.g. 'Study Description', 'Series Description', 'Body Part Examined' etc.). In some examples, similarly to as described above, each training text string may represent a concatenation of a plurality individual such text strings of different appropriate attributes of an image file. In any case, each training text string may be labelled with a body region to which the training text string corresponds. For example, in some examples, the training text string may be labelled with the actual body region represented by the medical imaging data of the file from which the training text strings originates, the actual body region being determined by an expert practitioner, for example. In some examples (as described in more detail below with reference to Figure 5), the training text string may be labelled with the body region determined to correspond to or be represented by the text string itself, as determined by an expert, for example. In either case, the body region label of each text string may be used as a supervisory signal in the training of the neural network.

In some examples, the trained neural network 406 may be configured to output the body region 408 in the form of a body region classification that a classifier portion of the trained neural network 406 has selected among a plurality of body region classifications for the input one or more text strings. For example, each classification may be a standardized word representing a body region, such as "ABDOMEN", "PELVIS", "CHEST" and the like. In such examples, each training text string may be labelled with the classification to which it belongs, and this may be used as a supervisory signal for training the neural network. For example, the training may comprise deep learning. For example, the training may comprise updating the weights of the connections between layers of neurons in the neural network so as to minimize the error between the classification predicted by the classifier for each of the training text strings and the actual classification of each of the training text strings as defined by their respective labels.

In some examples, the trained neural network 406 may be configured to output the body region 408 in the form of one or more numerical values, representing a region of a human body, that a regressor portion of the trained neural network 406 has calculated for the input one or more text strings 404. For example, the one or more numerical values may those of a 'body ruler', i.e. a ruler or scale defined for a human body where a value of 0.0 represents the tip of a human toe and a value of 1.0 represents the topmost portion of a human head (for example), with values in between 0 and 1 representing respective regions of the human body between the toe tip and the top of the head. In some examples, a two such numerical values may be used to represent a body region of the human body. For example, the two values may indicate the locations between which the body region is defined. For example, a bladder may be assigned the body ruler value [0.5, 0.55]. In such examples, each training text string may be labelled with the one (or more, e.g. two) numerical values representing the body region to which it corresponds, and this may be used as a supervisory signal for training the neural network 406. For example, the training may comprise deep learning. For example, the training may comprise updating the weights of the connections between layers of neurons in the neural network so as to minimize the error between the one (or more) numerical values predicted by the regressor for each of the training text strings and the actual one (or more) numerical values of each of the training text strings as defined by their respective labels.

Outputting the body region as one or more numerical values may allow for a precise and/or flexible determination of the body region represented by the medical imaging data of the file. For example, the numerical values are continuous and hence may be able to define a body region more precisely and with more flexibility for example as compared to use of a limited set of predefined classes. This may, in turn, allow for flexible and/or precise comparison of body regions, for example when selecting second medical imaging data as relevant to the first medical imaging data, as described in more detail below with reference to Figure 6.

In some examples, the trained neural network 406 may be a trained character-based neural network 406 configured to take as input individual characters of the obtained one or more text strings 404. In these examples, inputting the obtained one or more text strings 404 into the trained neural network 406 may comprise inputting individual characters of the obtained one or more text strings into the trained neural network 406. For example, the neural network 406 may comprise an encoder configured to encode each character of the input text string into a vector. For example, this may be done using a character embedding such as a 1-hot encoding for a vocabulary of characters including the alphabet, numerals 1 to 9, and special characters. These vectors may be used by the neural network 406 as a basis for determining the body region. Different architectures may be used.

For example, in some examples, the neural network 406 may comprise a character based Recurrent Neural Network (RNN), such as a Long Short-Term Memory (LSTM) RNN, such as a bidirectional LTSM. The vector for each character of the text string may be input sequentially into the RNN, after which the RNN will have a certain internal state (e.g. a vector representing the values of neurons thereof at the time when the vector for the last character of the text string is input) . This internal state may then be passed to the regressor or classifier of the neural network 406, which may then map the internal state onto a body region 408.

As another example, the neural network 406 may comprise a character based convolutional neural network (CNN). In these examples, the vectors for the successive characters of the text string may be assembled side by side to create a matrix. Convolutions and pooling operations may then be applied to the matrix to determine a condensed feature vector representing features present in the text string. The feature vector may then be passed to the regressor or classifier of the neural network 406, which may then map the feature vector onto a body region. The features by which the body region may be accurately mapped may themselves be learned during training of the neural network 406.

In some examples, other neural networks, e.g. word-based neural networks may be used. However, the neural network 406 comprising a character-based neural network may provide for determination of a body region that is robust with respect to abbreviations or misspellings or other words which were not part of the training data. For example, 'ABDOMEN' may be abbreviated as 'ABD', but since the first few letters are the same then the character-based neural network may generate a vector for these two words that are in a similar position in vector space (and hence appropriate body region may be determined), whereas a word based neural network may determine 'ABD' as out-of-vocabulary. This, in turn, may help provide an accurate determination, as out-of-vocabulary words can reduce accuracy.

As mentioned, each training text string may be labelled with a body region to which the training text string corresponds, and in some examples the training text string may be labelled with the body region determined to correspond to or be represented by the text string, as determined by an expert. As described with reference to Figure 5, in such cases, the training data set (comprising the training text strings and their labels) may be generated using a Graphical User Interface (GUI) 502. The GUI 502 may be configured to present one or more of the training text strings 508 and a representation of a human body 510 divided into selectable body regions 511 to a user. The GUI may be configured to, for each of the one or more presented training text strings 508, receive a user input selecting a body region 511 from the representation 510, and label the training text string 508 with a label 509 indicating the selected body region 511. In such a way a training data set comprising training text strings labelled with body region labels can be generated.

For example, referring to the specific example in Figure 5, the GUI 502 comprises a progress bar 504 which indicates to the user the particular pane of the GUI that is currently being displayed. The title of the particular pane is also shown 506, which in this case is "Annotate Key Words". In this example, the training text strings 508 are keywords extracted from attribute values of attributes of image files that store medical imaging data. In the illustrated example the keywords include 'aaa', 'ab', 'abdpel', 'abdroutine', 'aquired', 'aif', 'angiogram', and 'ascities'. The user is presented with a particular keyword (in this case 'aaa' which is in bold) and asked to select one of the displayed body regions 511 of the body representation 510 to which this corresponds. The user, e.g. being an expert and knowing that 'aaa' in the medical imaging context is an abbreviation for 'abdominal aortic aneurysm' selects the abdomen part of the displayed body representation 511, and accordingly the text string 'aaa' is labelled with the body region label 'ABDOMEN'. Similar selections may be made for the other presented keywords: 'ab'-'ABDOMEN', 'abdpel'- 'ABDOMEN', 'abdroutine'-'ABDOMEN', 'aif'-'HEAD', 'angiogram'-'CHEST', and 'ascities'-'ABDOMEN'. It is noted that the text string 'acquired' has not been labelled with a body region because it is not associated with any particular body region, and hence will not form part of the training data set. The GUI 502 also includes selectable buttons 512, namely 'Uterus', 'Prostate', 'Unknown' and 'None'. The 'Uterus' and 'Prostate' buttons are in order to provide a body region label of 'PROSTATE' and 'UTERUS' respectively. The 'Unknown' button is for if the user does not know the body region label but e.g. suspects it is capable of being assigned a body region label, and the 'None' button is for if the user knows that no body region is assignable to the text string (e.g. as was the case with 'acquired' above).

The GUI 502 may allow for a training data set comprising a plurality (e.g. 100s) of training text strings with the body region labels to be generated, in a simple and efficient manner. The labels are obtained using a representation (e.g. a stylized picture) of a human body, which is visual and simple for a user (including a medical professional and not necessarily a programming expert) to interact with. This simple and efficient interaction of the user with the GUI may in turn allow for the training data set to be generated (i.e. the text strings to be labeled) efficiently.

In the examples described above, the neural network 406 is trained to output a body region 408 based on the input text strings. In some examples, the neural network 406 may be further trained to output a laterality of the output body region 408 based on the input of the one or more text strings. The laterality may refer which side of the body (i.e. 'left' or 'right') the body region represented by the medical imaging data is located. For example, the output laterality may be either 'left' or 'right' as appropriate. In these examples, step 104 of the method of Figure 1 may further comprise determining the laterality of the body region represented by the medical imaging data based on the obtained output of the trained neural network 406. As one example, this may be by providing a dedicated laterality portion of the neural network 406 trained to output a laterality based on an input of the one or more text strings. For example, this may be trained using a training data set comprising training text strings each labelled with the laterality of the body region to which the text string corresponds. As another example, this may be by expanding a set of body region classifications onto which the neural network 406 maps the input text strings, to include 'left' and 'right' versions of each body region classification. For example, this may be trained using a training data set comprising training text strings each labelled with a body region and laterality classification to which the training text string corresponds. Determining the laterality of the body region may allow for a more precise body region to be determined, which may in turn allow for more accurate selection of relevant second medical imaging data, as described in more detail below.

As mentioned, in some examples the determined body region represented by the first medical imaging data may be used to select second medical imaging data relevant to (e.g. appropriate for comparison with) the first medical imaging data. For example, the body region may be determined for first medical imaging data of a current study for a given patient, and this may be used to select one or more sets of second medical imaging data (e.g. contained in one or more image files) of one or more previous studies for the given patient that are appropriate for comparison with the present study (e.g. same or similar body region). Referring now to Figure 6, there is illustrated a method of selecting second medical imaging data as relevant to the first medical imaging data.

The method comprises, in step 602, comparing a first body region 408 represented by first medical imaging data stored in the first image file 402 with each of a plurality of second body regions represented by a respective plurality of sets of second medical imaging data stored in a respective plurality of second image files. The method comprises, in step 604, selecting one or more of the sets of second medical imaging data as relevant to the first medical imaging data based on the comparison of the body regions.

Each of the second image files may be of the same type as the first image file 402. That is, each second image file further stores one or more attributes each having an attribute value comprising a text string indicating content of the second medical imaging data stored in the second image file. The first body region 408 may have been determined by applying steps 102 and 104 of the method described above with reference to Figure 1 to the one or more text strings of the first image file 402. Alternatively or additionally, at least one (and in some cases all) of the second body regions may be determined by applying steps 102 and 104 of the method described above with reference to Figure 1 to the one or more text strings of a respective at least one (and in some cases all) of the second image files.

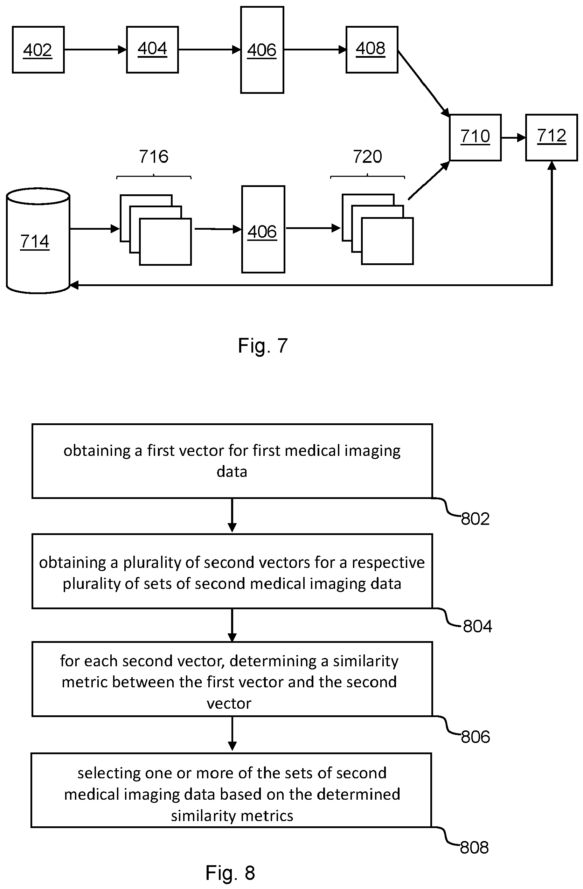

Figure 7 illustrates a flow between components of the method described above with reference to Figure 6, according to an example. Referring to Figure 7, in this example, similarly to as described above with reference to Figure 4, one or more of the text strings 404 from the first image file 402 are input into the trained neural network 406, and the trained neural network 406 outputs a first body region 408. The first body region 408 may be stored in association with an identifier of the first image file 402 (not shown).

Further, in this example, a plurality of one of more text strings 716 are extracted from a respective plurality of the second image files stored in the storage device 714 (e.g. of the same patient as the first image file 402). For example, each of the plurality of second image files may be from a different previous study of the patient. Each of the plurality of one or more text strings 716 of the second image files are input into the trained neural network 720, in turn, to obtain a respective plurality of second body regions 720. Each of the second body regions may be stored in association with an identifier of the associated second image file (not shown). As such, for example, the body region shown in image files of each of the plurality of previous studies may be determined. The first body region 408 may then be compared, by a comparator 710, with each of the plurality of second body regions 720, and a selection 712 of one or more of the sets of second medical imaging data may be made based on the comparison.

In some examples, the selection may be based only on the comparison. In other examples the selection may be based on further criteria as described in more detail below. In some examples, the selection may be a selection of the identifiers of the second image files in association with which the body regions are stored. The selected identifiers may be used to interrogate the storage device 714 and retrieve (e.g. prefetch) associated second image files from the storage device 714 (e.g. an online or near line DICOM archive device).

In some examples, the medical imaging data or image files of those studies (e.g. only those studies) including one of the one or more selected sets of second medical imaging data may be retrieved from the storage device 714. These files may be determined for example, by matching the 'study ID' attribute of the file containing the selected second medical imaging data. In some examples, as described in more detail below, the medical imaging data or image files of those series (e.g. only those series) including one or more of the selected sets of second medical imaging data may be retrieved from the storage device 714. These files may be determined for example, by matching the 'series ID' attribute of the file containing the selected second medical imaging data.

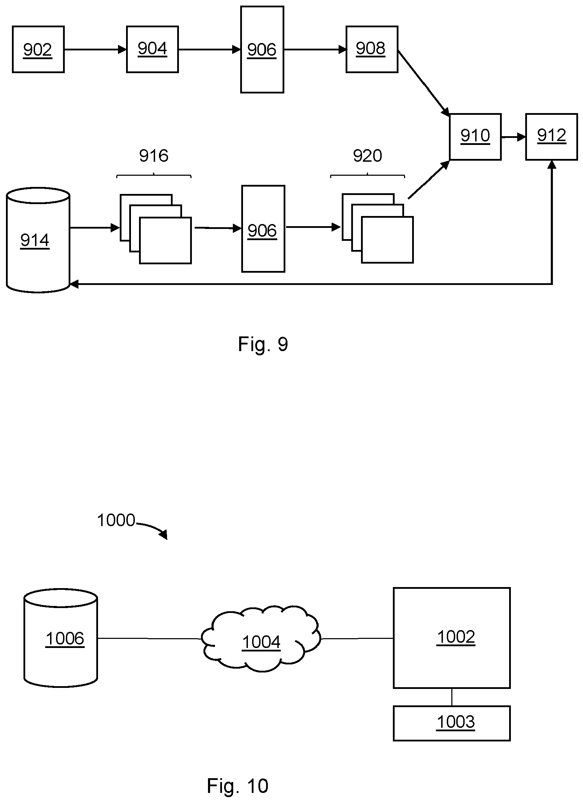

In any case, a rendering of one or more of the retrieved sets of second medical image data may be displayed on a display device (see e.g. 1003 of Figure 10).

In some examples, the first medical imaging data may represent a current medical image (or series of medical images) of a patient, and the plurality of sets of second medical imaging data may represent previous medical images (or series of medical images) of the patient. For example, the first image file may correspond to a current study of the patient, and each of the second image files may correspond to a respective different previous study of the patient. A medical professional may wish to compare one or more images of the current study with those of a relevant one of the previous studies of the patient, e.g. to assess the progression of a disease between the two studies. An important criterion enabling effective comparison is that the current and previous studies (i.e. the current and previous medical images thereof) be of the same or similar body part (e.g. head, abdomen, foot). By automatically selecting the second medical imaging data based on a comparison of the body regions determined for the first medical image data and the second medical imaging data, the method allows for the medical professional to compare e.g. the medical image of the current study with only relevant previous medical images (e.g. those of previous studies of the same or similar body region), which is efficient as compared to the user having to compare with all previous medical images.

Moreover, in some examples, the plurality of sets of second medical imaging data are stored in a remote storage device 714 (see also e.g. 1006 of Figure 10), which may be connected to the user's terminal (see e.g. 1002 of Figure 10) over a network (see e.g. 1004 of Figure 10). In these cases, the selected one or more sets of second medical imaging data (or sets of second medical imaging data of studies including the selected sets of second medical imaging data) may be retrieved from the remote storage device 714, 1006 (e.g. prefetched from a DICOM archive device 1006) without retrieving other ones of the plurality of sets of second medical imaging data. Since the body region may be determined based on text strings of attributes of the image files, the second medical imaging data of those files need only be retrieved from the remote storage 714, 1006 once they have been selected as relevant, and second medical imaging data not selected as relevant or to be retrieved need not be retrieved at all. As such, network resources may be efficiently deployed.

In some examples, the comparison of body regions may comprise determining whether the body region classification (e.g. 'ABDOMEN', 'CHEST') for the first medical imaging data is the same as that of the second medical imaging data. In some examples, if there is a match (e.g. both have the same body region classification 'ABDOMEN') then the second medical imaging data may be selected as relevant to the first medical imaging data.

In some examples, the comparison of body regions may comprise comparing the numerical values defining the body region for the first and second medical imaging data. For example, this may comprise determining whether the body region numerical value for the first medical imaging data is the same as or similar to or overlaps with that of the second medical imaging data. For example, if the numerical value for the first medical imaging data is 0.5, then a set of second medical imaging data having the numerical value 0.45 may be selected as being similar (e.g. differs by less than a predefined amount). As another example, if the numerical value for the first medical imaging data is [0.5, 0.55], then a set of second medical imaging data having the numerical value [0.5, 0.55] may be selected as it is the same, and a set of second medical imaging data having the numerical value [0.4, 0.55] may be selected as it is overlapping.

As mentioned, selecting one or more sets of second medical imaging data (e.g. previous studies of a given patient) as relevant to the first medical imaging data (e.g. a current study for the given patient) may be based on comparing the body regions thereof. However, in some examples, the selection may be based on further factors or criteria, as described in more detail below.

Beyond the current and previous studies showing the same body part, another important criterion enabling effective comparison of the images thereof is that the current and previous studies are of the same or similar modality (i.e. the mode or type of medical imaging used to capture the images thereof, e.g. CT, MRI, X-ray).

Accordingly, in some examples, the method may comprise determining, for each of the plurality of sets of second medical imaging data (e.g. of previous studies), an imaging modality relevance score between a first imaging modality of the first medical imaging data (e.g. of a current study) and a second imaging modality of the second medical imaging data (e.g. of a previous study). In these examples, selecting the one or more sets of second medical imaging data as relevant to the first medical imaging data may be further based on the determined imaging modality relevance score. For example, one of the sets of second medical imaging data (e.g. of one of the previous studies for a given patient) having a higher imaging modality relevance score may be selected for the first medical imaging data (e.g. of the current study for the patient) in preference to another of the sets of second medical imaging data (e.g. of another one of the previous studies for the given patient) having a lower imaging modality relevance score. In some examples, a set of second medical imaging data having the same body region as the first medical imaging data and having the highest imaging modality relevance score may be selected. In some examples, sets of second medical imaging data having the same or similar body region as the first medical image data may be pre-selected, for example as per the method described above with reference to Figure 6, and then one or more sets of second medical imaging data may be selected, from among the preselected sets, based on the imaging modality relevance score.

In some examples, the imaging modality used to capture the medical imaging data of a file may be determined from the attribute value of an imaging modality attribute of the image file in which the medical imaging data is stored. For example, the imaging modality may be obtained directly from the attribute value for the 'Modality' Attribute (e.g. identified by DICOM Tag (0008, 0060) of a DICOM file. The DICOM 'Modality' attribute value identifies the type of equipment that acquired the medical imaging data. In the DICOM standard, the values that can represent different imaging modalities are predefined. For example, it is defined that 'CT' represents 'Computed Tomography', and 'MR' represents 'Magnetic Resonance', and so on. Moreover, in some examples, the imaging modality attribute value may be set automatically to the appropriate value by the software being used with the equipment to generate the image file. Accordingly, the imaging modality may be reliably obtained directly from the 'modality' attribute of the file.

The imaging modality relevance score between two modalities may be a value representing the degree to which medical imaging data captured using one modality is relevant to (i.e. appropriate or useful for comparison with) medical imaging data captured using another modality. In some examples, the imaging modality relevance score may be determined using an imaging modality transition matrix. For example, each element of the matrix may correspond to one imaging modality relevance score between one specific imaging modality and another specific imaging modality. That is, the element sᵢⱼ of the matrix may be the imaging modality relevance score between the first (e.g. current) medical imaging data having imaging modality i and the second (e.g. prior) medical imaging data having the imaging modality j. For example, the imaging modality relevance score between i = MR and j = MR (i.e. s_{MRMR}) may be 0.6, whereas the imaging modality relevance score between i = MR and j = CT (i.e. s_{MRCT}) may be 0.22. In this case, for example if the first medical imaging data was captured using MR, then if two of the sets of second medical imaging both had the same body region as the first medical imaging data, but one set was captured using MR but another set was captured using CT, the set that was captured using MR may be selected in preference.

In some examples, the imaging modality relevance scores (i.e. each element sᵢⱼ of the transition matrix) may represent a probability that, given first medical imaging data associated with a particular first imaging modality i, a user (e.g. a medical professional) will select for comparison with the first medical imaging data second medical imaging data having a particular second imaging modality j. For example, this probability may be determined based on statistical analysis of logged user interaction with medical imaging data. For example, data logs may record the medical imaging files that have been retrieved for a given patient by a given medical professional in a given session. Statistical processing may be applied to these logs to determine the probabilities. For example, when reviewing a current medical imaging file whose modality is MR, if it is determined that medical professionals go on to review a previous medical imaging file of the patient whose imaging modality is MR 60% of the time, but goes on to review a previous medical imaging file of the patient whose imaging modality is CT 22% of the time, then s_{MRMR} may be determined as 0.6 and s_{MRCT} may be determined as 0.22. This may be done for all combinations of imaging modalities to populate the transition matrix. The imaging modality relevance scores (i.e. each element sᵢⱼ of the transition matrix) being based on statistical analysis of actual user interaction with the medical imaging data may help ensure that second medical imaging data of an appropriate modality is selected.

In examples described above, medical imaging data of a previous study for a given patient may be selected that is relevant to (e.g. appropriate for comparison with) medical imaging data of a current study for the given patient, e.g. based on body region and/or imaging modality. As mentioned, within a given study, there may be multiple series of medical images. For example, different series with a given study (specifically within a given modality, e.g. MR) may comprise medical imaging data that have been captured using different imaging parameters (e.g. for MR: Echo Time, Flip Angle, Echo Train Length, Patient Orientation etc.). Another important criterion enabling effective comparison of current and previous medical images by a medical professional may be that the medical imaging parameters used when capturing the current and previous medical images are the same or similar. Accordingly, in some examples, the selection of the second medical imaging data may alternatively or additionally be based on a comparison of imaging parameters. For example, second medical imaging data of a previous series within a previous study for a given patient may be selected as relevant to (e.g. appropriate for comparison with) first medical imaging data of a current series based on a comparison of the medical imaging parameters used to capture images of the previous and current series.

Referring to Figure 8, there is illustrated a method of selecting relevant medical imaging data based on imaging parameters.

In these examples, each of the first and second image files store one or more attributes each having an attribute value indicative of an imaging parameter used to capture the medical imaging data of the image file.

For example, similarly to as described above, each image file may be a DICOM file, and the one or more first attributes having attribute values indicative of an imaging parameter used to capture the medical imaging data thereof comprise one or more of the DICOM attributes 'Image Orientation Patient' (identified by DICOM Tag (0020, 0037) and whose value specifies the orientation cosines of the first row and first column of the medical imaging data with respect to the patient, an example value is '[1, 0, 0, 0, 1, 0]'); 'Series Description' (identified by DICOM Tag (0020, 0037) and whose value includes a description of the series, an example value is 'ax t1 whole pelvis' indicating an axial orientation); 'Echo Time' (identified by DICOM Tag (0018, 0081) and whose value specifies the time in millisecond between the middle of the excitation pule and the peak of the echo produced in MR imaging, an example value is '4.2'); 'Repetition Time' (identified by DICOM Tag (0018, 0080) and whose value specifies the time in milliseconds between the beginning of a pulse sequence and the beginning of the succeeding pulse sequence in MR imaging, and example value is '8'); 'Flip Angle' (identified by DICOM Tag (0018, 1314) and whose value specifies the steady state angle in degrees to which the magnetic vector is flipped from the magnetic vector of the primary field in MR imaging, an example value is '90'); 'Echo Train Length' (identified by DICOM attribute Tag (0018, 0091) and whose value specifies the number of lines in k-space (the array of numbers representing spatial frequencies in an MR image) acquired per excitation per image, an example value is '1'); 'Scanning Sequence' (identified by DICOM Tag (0018, 0020) and whose value indicates the type of MR data captured, an example value is 'SE' indicating spin echo type MR), 'Sequence Name' (identified by DICOM Tag (0018, 0024) and whose value species a user defined name for the combination of the scanning sequence and sequence variant in MR imaging, an example value is 'spcir_242'); and 'Protocol Name' (identified by DICOM Tag (0018, 1030) and whose value specifies the name of the CT protocol, a example value is 'T2W_TSE SENSE'). It will be appreciated that, in some examples, other such attributes, and indeed other types of image file, may be used.

In the example illustrated in Figure 8, the method comprises, in step 802, obtaining a first vector (see e.g. 908 of Figure 9) for the first medical imaging data, the first vector having been generated based on one or more of the attribute values (see e.g. 904 of Figure 9) indicative of an imaging parameter used to capture the first medical imaging data of the first image file (see e.g. 902 of Figure 9). The method comprises, in step 804, obtaining a plurality of second vectors (see e.g. 920 of Figure 9) for a respective plurality of the sets of second medical imaging data, wherein, for each set of second medical imaging data, the second vector 920 has been generated based on one or more of the attribute values (see e.g. 916 of Figure 9) indicative of imaging parameters used to capture the set of second medical imaging data of the second image file. The method comprises, in step 806, for each of the plurality of second vectors 920, determining a similarity metric indicative of the similarity between the first vector 908 and the second vector 920; and in step 808, selecting one or more (see e.g. 912 of Figure 9) of the sets of second medical imaging data as relevant to the first medical imaging data based on the determined similarity metrics. For example, in some examples, the set of second medical imaging data having the highest similarity metric among the plurality of sets of second medical imaging data may be selected. In some examples, the two or more sets of second medical imaging data having the highest two or more similarity metrics among the plurality of sets of second medical imaging data may be selected.

In some examples, the selection of the one or more sets of second medical imaging data (e.g. as described above with reference to Figures 1 to 7) may be further based on the determined similarity metrics, i.e. in addition to the comparison of the body region and/or the imaging modality relevance score described above with reference to Figures 1 to 7. For example, in some examples, second medical imaging data of a previous study (e.g. containing multiple series of second medical imaging data) may be pre-selected among previous studies for the patient as relevant to the current medical imaging data based on the comparison of body region and/or imaging modality as described above with reference to Figure 1 to 7; and second medical imaging data of a particular series, among the multiple series within the pre-selected study, may be selected as relevant to the current medical imaging data based on the determined similarity metrics as described with reference to Figure 8. In this case, the one or more sets of second medical imaging data (e.g. representing one or more medical images of a given series) may be selected as relevant to the first medical imaging data based on a comparison of the body regions, the imaging modality relevance score, and the determined similarity metric.

However, in certain other examples the method described with reference to Figure 8 may be applied independently of the methods described above with reference to Figures 1 to 7. For example, the selection of the one or more sets of second medical imaging data may be based on the determined similarity metrics and need not necessarily be based additionally the on comparison of the body region and/or the imaging modality relevance score described above with reference to Figures 1 to 7. For example, an appropriate previous study may have already been determined by other means, and the method of Figure 8 may be applied to the image files of this previous study to select the second medical imaging data of a particular series within the previous study as relevant to the current medical imaging data.

In either case, the method described with reference to Figure 8 may allow for one or more sets of second medical imaging data (e.g. representing a certain series of previous medical images) that were captured using the same or similar imaging parameters as the first medical imaging data (e.g. representing a current medical image) to be automatically selected. This may provide for efficient selection of previous medical images appropriate for comparison with a current medical image, for example as compared to opening and assessing all of the previous medical images for the patient. Moreover, the selection being based on attribute values (which are relatively small in size) of the image files allows for selection without having to extract or analyze the medical imaging data itself (which is relatively large in size), and hence may allow for efficient selection. Moreover, the selection being based on vectors generated based on the attribute values may allow for a flexible selection. For example, the comparison of vectors in the feature space may be more robust and/or flexible with respect to non-exact matches between parameters, e.g. as compared to attempting to match the imaging parameters directly.

Figure 9 illustrates a flow between components of the method described with reference to Figure 8, according to an example. A first image file 902 stores the first medical imaging data and attributes having first attribute values indicative of imaging parameters used to capture the first medical imaging data. These first attribute values 904 are extracted from the first image file 902 and provided to a vectorizer 906, and the vectorizer outputs the first vector 908. The plurality of second image files are stored in a storage 914. In some examples, each of the plurality of second image files may be from a respective different series within a study. A plurality of sets of one or more second attribute values 916 (each indicating imaging parameters used to capture the second medical imaging data stored in the respective second file in which they are included) are extracted from a respective plurality of the second image files. These sets of second attribute values 916 are provided in turn to the vectorizer 906, which outputs a respective plurality of the second vectors 920 (one of each of the input sets of one or more second attribute values). The first vector 908 and/or the second vector 920 may be stored in association with the first/second image file (or an identifier thereof) for which it was generated. The first vector 908 and each of the plurality of second vectors 920 are input to a comparator 910 which determines the similarity metric between the first vector and each of the second vectors. The comparator 910 may output a selection 912 of one or more of the sets of second medical imaging data (or identifiers thereof) based on the determined similarity metrics (e.g. the one or more with the highest similarity metrics may be selected). The selected one or more sets of second medical imaging data may then be retrieved from the storage 914. In some examples, as mentioned above, second medical imaging data of series (e.g. only of series) containing one of the selected second sets may be retrieved from the storage 914. In other words, in some examples, sets of second medical imaging data (e.g. only sets of second medical imaging data) that are included in a series including one or more of the selected sets of second medical imaging data may be retrieved from the storage 914.

In some examples, as already mentioned, the plurality of sets of second medical imaging data may be stored in a remote storage (see e.g. 1006 of Figure 10). In these examples, the method may comprise retrieving the selected one or more sets of second medical imaging data (or sets of medical imaging data of series including the selected sets of second medical imaging data) from the remote storage without retrieving other ones of the plurality of sets of second medical imaging data. This may provide for efficient use of network resources.

In some examples, the method may comprise generating display data to cause a display device (see e.g. 1003) to display a rendering of the first medical imaging data and a rendering of one or more of the selected (or otherwise retrieved) sets of second medical imaging data. This may provide for a medical professional to visually assess the differences between the first (e.g. current) and second (e.g. previous) medical imaging data for a patient. Since the second (previous) medical imaging data has been selected to be appropriate for comparison with the first (current) medical imaging data, the user can better focus on differences due to a progression of disease represented thereby, rather than differences due to non-disease related factors such as modality and imaging parameters.

In some examples, the method may comprise generating the first vector 908 and/or one or more (for example all) of the second vectors 920.

In examples where one or more of the first attribute values 904 or second attribute values 916 comprises a text string, generating the first vector 908 or the second vector 920, respectively, may comprise encoding the text string into a vector representation. For example, the text string may include words which may be encoded into a vector representation using word embeddings. For example, word embeddings map words of a dictionary onto vector space, where the words in the dictionary and the vectors for each word may be generated by applying a word embedding model to a corpus of training text. An example of a known word embedding model is "Word2vec" which uses a neural network to learn word embeddings from a corpus of training text. In some examples, pre-trained word embeddings, which for example have been pre-trained on a vast corpus of generic text, may be used. In some examples, the training text may comprise medical text such as radiology reports, medical literature and/or the text strings of attribute values of training image files. This may allow for the semantic meaning (within the context of the training text) of words and abbreviations specific to the medical field to be learned. Where the text string comprises multiple words, the vector from the word embedding for each word may be combined to generate the first vector (or part of the first vector), e.g. by concatenating, or taking an average of, the vector of the word embedding for each word of the text string. Other methods may be used.

In examples where one or more of the first attribute values 904 or second attribute values 906 comprises numerical values, generating the first vector 908 or the second vector 920, respectively, may comprise formatting the numerical values into a vector representation. For example, one or more of the attribute values may be a numerical value. For example, as described above, an example value of the example DICOM attribute 'Echo Time' is '4.2'. In such examples, the attribute value may be used as an element of the first or second vector as appropriate. In some examples, the attribute value may be normalized before including it as an element of the first or second vector as appropriate. For example, the echo time attribute value may be divided by 10000 before being included as an element of the vector. As another example, one or more of the attribute values may include a plurality of numerical values, e.g. a series of values. In such examples, the series of numerical values may be formatted into a column vector with one numerical value per element. For example, as described above, an example attribute value of the example DICOM attribute 'Image Orientation Patient' is '[1, 0, 0, 0, 1, 0]'. This may be formatted into a column vector, and used as the first or second vector (or a part thereof) as appropriate.

In some examples, generating the first vector 908 or the second vector 920 comprises, for each of a plurality of the first attribute values 904 or second attribute values 916, respectively, generating a third vector based on the first attribute value or second attribute value, respectively, and combining the third vectors to generate the first vector 908 or the second vector 920, respectively. For example, the plurality of attribute values may be 'Series Description', 'Echo Time' and 'Image Orientation Patient'. In this case, a third vector may be generated for each of these three attribute values as described above, e.g. v_{SD} v_{ET} v_{IOP}, respectively. Then, each of these three third vectors may be concatenated to generate the first vector 908 or second vector 920 as appropriate, e.g. [v_{SD}, v_{ET}, v_{IOP}].

In some examples, determining the similarity metric may comprise, for each second vector 920, determining the Cosine similarity between the first vector 908 and the second vector 920. As is known, the Cosine similarity is the dot product between two vectors, and represents the similarity between the two vectors. For example, a Cosine similarity of 1 indicates the same or highly similar vectors (and hence which correspond to the same or highly similar imaging parameters) and a Cosine similarity of 0 indicates orthogonal vectors (and hence which correspond to diametrically opposed or highly dissimilar imaging parameters). In some examples a set of second medical imaging data having a second vector 920 with a high cosine similarity with a first vector 908 of first medical imaging data is selected in preference to a set of second medical imaging data having a second vector 920 with a low cosine similarity. In some examples, other similarity metrics may be used, such as Euclidean distance between the first vector 908 and the second vector 920. In these examples, the comparator 910 may comprise a calculation unit (not shown) that calculates the Cosine similarity (or other such similarity measure) between the first and second vectors.

In some examples, the comparator 910 may comprise a neural network (not shown) trained to output a value indicative of the similarity between two vectors based on an input of the two vectors. In some examples, determining the similarity metric may comprise, for each of the second vectors 920, inputting the first vector 908 and the second vector 920 into the trained neural network; and obtaining the output from the trained neural network thereby to determine a value indicative of the similarity between the first vector and the second vector. For example, the neural network may be a deep neural network comprising one or more hidden layers of neurons between initial and final layers. The initial layer may be configured to take as input the first vector and the second vector of a fixed size. The neural network may comprise a regressor (not shown) configured to map a vector representation from a final layer of the neural network to a value (e.g. between 0 and 1) indicating the similarity between two input vectors.

In some examples, the neural network may be trained based on a training data set, the training data set comprising a plurality of pairs of such vectors (e.g. a plurality of pairs of vectors where each vector of the pair has the same format as the first vector 908 and the second vector 908, respectively), each pair being labelled with a training similarity value, the training similarity value providing a supervisory signal during training of the neural network. In practice, the training data set may comprise 100s or 1000s of such labelled training pairs. For example, the training may comprise deep learning. For example, the training may comprise updating the weights of the connections between layers of neurons in the neural network so as to minimize the error between the similarity value predicted by the regressor for each of the training pairs of vectors and the actual similarity value of each of the training pairs as defined by their respective labels.

In some examples, the training similarity value label may, for each of the training pairs of vectors, represent a probability that, given first medical imaging data having particular first attribute values represented in one vector of the training pair, a user will select for comparison with the first medical imaging data second medical imaging data having particular second attribute values represented in the other vector of the pair. For example, this probability may be determined based on statistical analysis of logged user interaction with medical imaging data. For example, data logs may record the medical imaging files that have been retrieved for a given patient by a given medical professional in a given session. Statistical processing may be applied to these logs to determine the probabilities. For example, for current medical imaging files whose imaging parameters are X, it may be determined what percentage of the time medical professionals go on to review previous medical imaging files whose imaging parameters are Y (e.g. 60%) and what percentage of the time medical professionals go on to review previous medical imaging files whose imaging parameters are Z (e.g. 20%). In this case, two training pairs may be generated, one having the vectors representing X and Y and having the training similarity value label of 0.6, and another having the vectors representing X and Z and having the training similarity value label of 0.2. This may be done for a large number of combinations of imaging parameters to generate the training data set. The training data set being based on statistical analysis of actual user interaction with the medical imaging data may help ensure that second medical imaging data that was captured using appropriate imaging parameters is selected.

Referring to Figure 10, there is illustrated an example system 1000 in which the method according to any one or combination of the examples described above with reference to Figures 1 to 9 may be implemented in some examples. The system 1000 comprises a computer 1002, a display device 1003, a network 1004 and a storage 1006. The computer 1002 may be configured to perform the method according to any one or combination of the examples described above with reference to Figures 1 to 9. The display device 1003 may be used to display the renderings (not shown) of the medical imaging data described above, and/or the GUI 502 described above with reference to Figure 5. The computer 1002 is configured to communicate with the storage 1006 over the network 1004. For example, the network 804 may comprise a Local Area Network or a Wide Area Network, such as the Internet. In this example, the storage 1006 represents an archive, e.g. a DICOM archive device, and is configured to store the imaging files according to any of the examples described above. For example, the storage 1007 may implement the storage 714 described above with reference to Figure 7 and/or the storage 914 described above with reference to Figure 9. For example, the storage 914 may store the first image files 402, 902 and/or the second image files. The storage 1006 is remote from the computer 1002 in the sense that it is accessible via the network 1004 rather than locally at the computer 802. In such implementation environments, it can be important to reduce or limit the volume of data that is transmitted over the network 1004, for example due to limited bandwidth resources. Medical imaging data can be particularly large in terms of size, so it can be important to reduce the transmission of medical imaging data over the network 1004, where possible. Rather than extracting all second (e.g. previous) medical imaging data for a patient for a user to compare with first (e.g. current) medical imaging data, according to examples of the present disclosure, as mentioned above the computer 1002 may select one or more sets of second (e.g. previous) medical imaging data that are relevant to the first (e.g. current) medical imaging data (e.g. is of a study of the same or similar body region and/or using the same or similar modality; and/or is of a series using the same or similar imaging parameters) based on attribute values of attributes of the files in which the medical imaging data are stored, and hence may extract only those sets of second (e.g. previous) medical imaging data that are relevant for the comparison. Accordingly, the overall volume of data that is transmitted over the network 1004 may be reduced, and network resources deployed more efficiently.

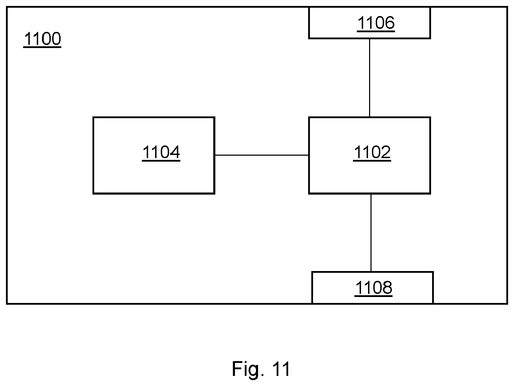

Referring to Figure 11, there is illustrated an apparatus 1100 according to an example. The apparatus may be a computer 1100. The apparatus 1100 may be used in place of the computer 1002 described above with reference to Figure 10. The apparatus 1100 may be configured to perform the method according to any one or combination of the examples described above with reference to Figures 1 to 10. The apparatus 1100 may be configured for communication with a remote storage 1006 over a network 1004, for example according to any of the examples described above with reference to Figures 1 to 10.

As illustrated, the apparatus 1100 comprises a processor 1102, a memory 1004, an input interface 1006 and an output interface 1108. The memory 1104 may store instructions, which when executed by the processor 1102, cause the apparatus 1100 to perform the method according to any one or combination of the examples described above with reference to Figures 1 to 10. The instructions may be provided on a non-transitory computer readable medium. The instructions may be in the form of a computer program. The input interface 1106 may, for example, be configured to receive the user inputs from the GUI 502 described above with reference to Figure 5. For example, the input interface 1106 may be connected to an input means such a keyboard and/or mouse (not shown), via which the user/radiologist may provide input and/or selections. The processor 1102 may be configured to receive the inputs and/or selections via the input interface 1106. The input interface 1106 may alternatively or additionally be configured to receive the image files, medical imaging data, text strings, attribute values, vectors, and/or training data sets according to any one of the examples described above with reference to Figures 1 to 10. The output interface 1108 may be configured to output the determined body region, the selected sets of one or more second medical imaging data, and/or the display data according to any one of the examples described above with reference to Figures 1 to 10. The output interface 1108 may be in communication with the remote storage 1006 and transmit signals to the remote storage 1006 to cause retrieval of the selected second medical image data (of files thereof, or files or data of the study or series of which the selected medical imaging data is part) . Alternatively or additionally, for example, the output interface 1108 may be connected to a display device, such as the display device 1003 of Figure 10, and output display data to the display device. For example, the display device may be caused, via the output display data, to display the renderings of the medical imaging data and/or the GUI 502 according to any one or combination of the examples described above with reference to Figures 1 to 10. The processor 1102 may be configured to output the display data via the output interface 1108.

As mentioned above, in certain examples the method described with reference to Figures 8 and 9 may be used independently of the method described with reference to Figures 1 to 7. For example, the selection of the one or more sets of second medical imaging data may be based on the determined similarity metrics and need not necessarily be based additionally the on comparison of the body region and/or the imaging modality relevance score described above with reference to Figures 1 to 7. Aspects of these certain examples are described in the following numbered clauses:
A1. A computer implemented method for determining a relevant medical imaging data set, the method comprising:
   obtaining a first vector for first medical imaging data, the first vector having been generated based on one or more first attribute values of attributes of a first image file further comprising the first medical imaging data, each first attribute value being indicative of an imaging parameter used to capture the first medical imaging data;
   obtaining a plurality of second vectors for a respective plurality of sets of second medical imaging data, wherein, for each set of second medical imaging data, the second vector has been generated based on one or more second attribute values of attributes of a second image file comprising the second medical imaging data, the one or more second attribute values being indicative of an imaging parameter used to capture the second medical imaging data;
   for each of the plurality of second vectors, determining a similarity metric indicative of the similarity between the first vector and the second vector; and
   selecting one or more of the sets of second medical imaging data as relevant to the first medical imaging data based on the determined similarity metrics.
A2. The computer implemented method according to clause A1, wherein the plurality of sets of second medical imaging data are stored in a remote storage, and the method comprises:
   retrieving the selected one or more sets of second medical imaging data, or sets of second medical imaging data of series including the selected one or more sets of second medical imaging data, from the remote storage without retrieving other ones of the plurality of sets of second medical imaging data.
A3. The computer implemented method according to clause A1 or clause A2, wherein the method comprises:
   generating display data to cause a display device to display a rendering of the first medical imaging data and a rendering of one or more of the selected or retrieved sets of second medical imaging data.
A4. The computer implemented method according to any one of clause A1 to clause A3, wherein the method comprises:
   generating the first vector and/or one or more of the second vectors.
A5. The computer implemented method according to clause A4, wherein, where one or more of the first attribute values or second attribute values comprises a text string, generating the first vector or the second vector, respectively, comprises encoding the text string into a vector representation.
A6. The computer implemented method according to clause A4 or clause A5, wherein, where one or more of the first attribute values or second attribute values comprises numerical values, generating the first or the second vector, respectively, comprises formatting the numerical values into a vector representation.
A7. The computer implemented method according to any one of clause A4 to clause A6, wherein generating the first vector or the second vector comprises: for each of a plurality of the first attribute values or second attribute values, respectively, generating a third vector based on the first attribute value or second attribute value, respectively; and combining the third vectors to generate the first vector or the second vector, respectively.
A8. The computer implemented method according to any one of clause A1 to clause A7, wherein, for each of the plurality of second vectors, determining the similarity metric comprises determining the Cosine similarity between the first vector and the second vector.
A9. The computer implemented method according to any one of clause A1 to clause A8, wherein, for each of the plurality of second vectors, determining the similarity metric comprises:
   inputting the first vector and the second vector into a trained neural network, the neural network having been trained to output a value indicative of the similarity between two such vectors based on an input of the two vectors;, and obtaining the output from the trained neural network thereby to determine a value indicative of the similarity between the first vector and the second vector. A10. The computer implemented method according to clause A9, wherein the neural network has been trained based on a training data set, the training data set comprising a plurality of pairs of such vectors, each pair being labelled with a training similarity value, the training similarity value providing a supervisory signal during training of the neural network.
A11. The computer implemented method according to clause A10, wherein for each of the pairs of vectors, the similarity value label represents a probability, determined based on statistical analysis of logged user interaction with medical imaging data sets, that given first medical imaging data having particular first attribute values represented in one vector of the pair, a user will select for comparison with the first medical imaging data second medical imaging data having particular second attribute values represented in the other vector of the pair.
A12. The computer implemented method according to any one of clause A1 to clause A11, wherein selecting one or more of the sets of second medical imaging data as relevant to the first medical imaging data is further based on one or more of:
   a comparison of a first body region represented by the first medical imaging data with each of a plurality of second body regions represented by the plurality of sets of second medical imaging data; and
   an imaging modality relevance score determined between a first imaging modality of the first medical imaging data and a second imaging modality of the second medical imaging data.
A13. The computer implemented method according to any one of clause A1 to clause A12, wherein the or each image file is a DICOM file, and the one or more attributes having attribute values indicative of an imaging parameter used to capture the medical imaging data set thereof comprise one or more of the DICOM attributes 'Image Orientation Patient', 'Series Description', 'Echo Time', 'Repetition Time', 'Flip Angle', 'Echo Train Length', 'Scanning Sequence', 'Sequence Name', and 'Protocol Name'.
A14. Apparatus configured to perform the method according to any one of clause A1 to clause A13.
A15. A computer program which, when executed by a computer, causes the computer to perform the method according to any one of clause A1 to clause A13.

The above examples are to be understood as illustrative examples of the invention. It is to be understood that any feature described in relation to any one example may be used alone, or in combination with other features described, and may also be used in combination with one or more features of any other of the examples, or any combination of any other of the examples. Furthermore, equivalents and modifications not described above may also be employed without departing from the scope of the invention, which is defined in the accompanying claims.

## Claims

1. A computer implemented method of determining a body region (408) represented by medical imaging data (204, 316) stored in a first image file (200, 300, 402), the first image file further storing one or more attributes (202, 310) each having an attribute value (206, 314) comprising a text string (404) indicating content of the medical imaging data, the method comprising:
(a) obtaining (102) one or more of the text strings of the first image file;
(b) inputting (104) the obtained one or more text strings into a trained machine learning model (406), the machine learning model having been trained to output a body region based on an input of one or more such text strings, and obtaining the output (408) from the trained machine learning model thereby to determine the body region represented by the medical imaging data.

2. The computer implemented method according to claim 1, wherein the method comprises:
comparing (602) a first body region (408) represented by first medical imaging data stored in the first image file (402) with each of a plurality of second body regions (720) represented by a respective plurality of sets of second medical imaging data stored in a respective plurality of second image files, each second image file further storing one or more attributes each having an attribute value comprising a text string (716) indicating content of the second medical imaging data stored in the second image file; and
selecting (604) one or more of the sets of second medical imaging data as relevant to the first medical imaging data based on the comparison of the body regions;
wherein the first body region is determined by applying steps (a) and (b) to the one or more text strings of the first image file; and/or
wherein at least one of the second body regions is determined by applying steps (a) and (b) to the one or more text strings of a respective at least one of the second image files.

3. The computer implemented method according to claim 2, wherein the plurality of sets of second medical imaging data are stored in a remote storage device (714, 914, 1006), and the method comprises:
retrieving the selected one or more sets of second medical imaging data, or sets of second medical imaging data of studies including the selected one or more sets of second medical imaging data, from the remote storage device without retrieving other ones of the plurality of sets of second medical imaging data.

4. The computer implemented method according to claim 2 or claim 3, wherein the method comprises:
generating display data to cause a display device (1003) to display a rendering of the first medical imaging data and a rendering of one or more of the selected or retrieved sets of second medical imaging data.

5. The computer implemented method according to any one of claim 2 to claim 4, wherein the method comprises:
determining, for each of the plurality of sets of second medical imaging data, an imaging modality relevance score between a first imaging modality of the first medical imaging data and a second imaging modality of the second medical imaging data; and
wherein selecting the one or more sets of second medical imaging data as relevant to the first medical imaging data is further based on the determined imaging modality relevance score.

6. The computer implemented method according to claim 5, wherein the imaging modality relevance score is determined using an imaging modality transition matrix, wherein each element of the imaging modality transition matrix represents a respective probability, determined based on statistical analysis of logged user interaction with medical imaging data, that given first medical imaging data associated with a particular first imaging modality a user will select for comparison with the first medical imaging data second medical imaging data having a particular second imaging modality.

7. The computer implemented method according to any one of claim 2 to claim 6, wherein each of the first and second image files stores one or more attributes each having an attribute value (904, 916) indicative of an imaging parameter used to capture the medical imaging data of the image file, wherein the method comprises:
for each of a plurality of sets of the second medical imaging data, determining a similarity metric indicative of the similarity between a first vector (908), generated based on one or more of the attribute values (904, 916) indicative of an imaging parameter used to capture the first medical imaging data, and a second vector (920) generated based on one or more of the attribute values (916) indicative of an imaging parameter used to capture the second medical imaging data; and
wherein selecting the one or more sets of second medical imaging data as relevant to the first medical imaging data is further based on the determined similarity metric.

8. The computer implemented method according to any one of claim 1 to claim 7, wherein the trained machine learning model is a trained neural network.

9. The computer implemented method according to claim 8, wherein the trained neural network (406) comprises a trained character-based neural network configured to take as input individual characters of the obtained one or more text strings, whereby inputting the obtained one or more text strings into the trained neural network comprises inputting individual characters of the obtained one or more text strings into the trained character-based neural network.

10. The computer implemented method according to claim 8 or claim 9, wherein the trained neural network is configured to output the body region in the form of one or more numerical values, representing a region of a human body, that a regressor portion of the trained neural network has calculated for the input one or more text strings.

11. The computer implemented method according to any one of claim 8 to claim 10, wherein the neural network has been trained using a training data set comprising a plurality of training text strings, each training text string being from an attribute value of an attribute of an image file and indicating content of medical imaging data further stored in the image file, each of the plurality of training text strings being labelled with a body region to which the training text string corresponds, the label being used a supervisory signal in the training of the neural network.

12. The computer implemented method according to claim 11, wherein the training data set has been generated using a Graphical User Interface (502), GUI, configured to: present one or more of the training text strings (508) and a representation of a human body (510) divided into selectable body regions (511) to a user; and for each of the one or more presented training text strings, receive a user input selecting a body region from the representation, and label the training text string with a label (509) indicating the selected body region.

13. The computer implemented method according to any one of claim 1 to claim 12, wherein the or each of the image files is a DICOM file (300), and the one or more attributes having attribute values comprising text strings indicating content of the medical imaging data thereof comprise one or more of the DICOM attributes 'Study Description', 'Series Description' and 'Body Part Examined'.

14. Apparatus (1002, 1100) configured to perform the method according to any one of claim 1 to claim 13.

15. A computer program which, when executed by a computer (1002, 1100), causes the computer to perform the method according to any one of claim 1 to claim 13.
